(19) Europäisches Patentamt
European Patent Office
Office européen des brevets

(11) Publication number: **0 135 729**
**A1**

(12) # EUROPEAN PATENT APPLICATION

(21) Application number: **84109159.8**

(22) Date of filing: **02.08.84**

(51) Int. Cl.⁴: **C 07 C 9/04**, C 07 C 4/06, C 07 C 1/20, B 01 J 23/74, B 01 J 23/94

(30) Priority: **04.08.83 GB 8321086**

(43) Date of publication of application: **03.04.85 Bulletin 85/14**

(84) Designated Contracting States: **AT BE DE FR GB IT NL**

(71) Applicant: **VEG-GASINSTITUUT N.V., Wilmersdorf 50, NL-7327 AC Apeldoorn (NL)**

(72) Inventor: **Kuijpers, Eugene Gerard Marie, Dr., Topaasstraat 15, NL-7314 HS Apeldoorn (NL)**
Inventor: **Geus, John Wilhelm, Gezichtslaan 100, NL-3723 GJ Bilthoven (NL)**
Inventor: **de Bokx, Pieter Klaas, Jadelaan 76, NL-3523 CW Utrecht (NL)**

(74) Representative: **Türk, Dietmar, Dr. rer. nat. et al, Redies, Redies, Türk & Gille Patentanwälte Brucknerstrasse 20, D-4000 Düsseldorf 13 (DE)**

(54) **Process for the production of methane rich gases, a regenerated nickel catalyst useful therefor and process for preparing it.**

(57) The invention relates to a process for the production of methane rich gases by the conversion of a mixture comprising hydrocarbons, steam, and optionally methanol or methanol and optionally steam by using nickel catalysts on a thermally stable oxidic carrier, characterized in that the metallic nickel particles are chemically bonded to the thermally stable oxidic carrier. The invention also relates to a process for regenerating a nickel catalyst for use in such process, and a regenerated nickel catalyst obtainable by such regeneration process. Furthermore, the invention relates to the use of such reactivated nickel catalyst for the above process.

EP 0 135 729 A1

2. Juli 1984

# PROCESS FOR THE PRODUCTION OF METHANE RICH GASES, A REGENERATED NICKEL CATALYST USEFUL THEREFOR AND PROCESS FOR PREPARING IT.

## BACKGROUND OF THE INVENTION

The invention relates to a process for the production of methane rich gases by the conversion of a mixture comprising hydrocarbons, steam and optionally methanol or methanol and optionally steam by using nickel catalysts on a thermally stable oxidic carrier. The invention also relates to a regenerated nickel catalyst useful for such process, a process for preparing such catalyst and the use thereof for the process for the production of methane rich gases. Processes of this type have been developed by the British Gas Corp. and its predecessors (the Gas Council, the North-Western Gas Board) and later by Heinrich Koppers GmbH, LURGI Metallgesellschaft AG, and Japan Gasoline Corp. Catalysts for these processes have been developed amongst others by ICI Ltd., BASF and Nikki Chemical Company. All theses processes, most of them described in patent specifications (DE-B-1545463; US-A-4182926; 4140493; GB-A-1437957; US-A-3420642; DE-B-1227603; GB-A-1265481; 1443277) have two things in common:

1. Mixtures of hydrocarbons are converted with steam in a temperature range between 350 and 550 $^{o}$ C and at pressures roughly between 0.5 and 2.5 MPa into a gas mixture containing mostly methane and also hydrogen and carbon dioxide and small quantities of carbon monoxide.

2. Catalysts for these processes are mostly based on nickel as the active component.

For an conomically favourable utilisation of the process it would be attractive to keep the steam addition to the hydrocarbon feed at the level that is minimally required to stay outside the carbon deposition area. Furthermore, it would be desirable to have a great flexibility in the hydrocarbon feedstock composition. It is well-known, however, that variations in the feedstock composition and "near thermodynamic" steam to hydrocarbon ratios when operating the process, will give problems because of catalyst deactivation, carbon deposition and consequently an increased pressure drop over the reactor with incomplete conversion of the hydrocarbon feed. The properties of the catalyst greatly influence the behaviour of the process when pushing its operating towards the thermodynamic limits for carbon deposition.

Thus, a great deal of work has been devoted to the search of good catalysts for this process. Also, process operating procedures can improve the overall performance either as such or as a consequence of the improved properties of the catalyst. In the many publications on this subject it is explained that the reaction comprises several steps, the most important of which are a first step in which the hydrocarbons decompose onto the nickel surface releasing hydrogen into the gas phase while carbon rich intermediates are left on the catalyst surface, this reaction is endothermic; and a second step in which the carbon rich species react with steam to form methane,

 

this reaction is exothermic. The first reaction causes carbonacious material to be formed on the catalyst at the entrance of the catalyst bed. As a consequence there is a temperature drop in this part of the catalyst bed and also a progressive deactivation. By an increased steam addition and by an increased entrance temperature the reaction between steam and carbonacious deposits can be promoted leading to a retardation of catalyst deactiviation. Obviously, this will reduce the efficiency of the process. Furthermore, because of the consequently larger gas throughput, the reactor volume must be increased to maintain a certain space velocity. Therefore, many efforts in catalyst research were directed towards enhancing the reaction between steam and carbonacious deposits.

A number of nickel catalysts may be used, examples of which can be found in DE-B-1 199 427 and 1 227 603.

BE-A-634 920 describes catalysts which are suitable for reforming hydrocarbons with steam and contain nickel or cobalt in the form of the metal or in the form of a compound which may be reduced to form the metal as well as a metal of the platinum group upon a refractory support such as alumina. These catalysts may also contain a small amount of an alkali metal or an earth alkali metal in order to prevent the deposition of carbon upon the catalyst. It is also known from said Belgian patent specification 634 920 that by carrying out the reforming of hydrocarbons having a boiling point of at most 350° C with steam at a

temperature of 600-1000° C synthesis gas or a gas having a low methane content are obtained but that by carrying out said processes at a temperature of 450-700° C a methane-rich gas is obtained.

According to FR-B-1 394 202 the life of the catalysts in the process as described in DE-B-1 180 481 can considerably be increased by recycling hot reaction gases containing water vapour through the catalyst bed, mixed with the hydrocarbon vapour and steam being supplied to said bed.

US-A-4 140 493 describes a nickel catalyst with calcium phosphate support and barium or uranium as a promotor. According to Canadian patent 848 888 the addition of alkali metals to nickel catalysts enables the utilisation of heavier hydrocarbon feedstocks.

A more recent development in this field is described in US-A-4 216 123.

Here the problem is solved by influencing the occurrence of the endothermal and exothermal reactions as a function of the displacement along the catalyst bed in the reactor. The catalyst is characterized by the fact that it consists of a group VIII metal from the periodic system with alumina and optionally minor amounts of alcali or alcaline-earth metals with a very specific pore size distribution. This specific pore size distribution invokes a split up to the consecutive reactions in the process so as to give a good balance between

the endothermal and exothermal process steps. Although this process has very favourable properties it also has some disadvantages. The most important is that by virtue of the conversion mechanism the overall conversion is limited by diffusion of higher hydrocarbons into the pores which makes the catalyst relatively low active. This can only be overcome by using large catalyst volumes and consequently large reactor sizes which is obviously not economically attractive.

## SUMMARY OF THE INVENTION

The present invention is aimed at mainly 4 objectives.

1. Finding a catalyst which will convert hydrocarbon steam mixtures at near carbon deposition boundary conditions into a methane rich gas without substantial deactivation.

2. Finding a catalyst which will convert hydrocarbon feedstocks with considerable variation in composition.

3. Finding a catalyst which is already active at relative low temperature in promoting the above reactions.

4. Finding a catalyst which can be reactivated and which can be used in reactivated form after it lost its original activity.

Furthermore, finding a catalyst that allows a large temperature difference over the catalyst bed would be advantageous.

In the process according to the state of the art, the temperature difference over the catalyst bed is mostly limited to below $80^o$ because of catalyst requirements. This is effectuated by the addition of excess steam with - obviously - the same disadvantages as mentioned before.

The conversion of methanol into a methane-rich gas proceeds roughly according to the reaction:

$$4\,CH_3OH \longrightarrow 3\,CH_4 + 2\,H_2O + CO_2$$

Thus, theoretically, no steam has to be added for the catalytic conversion of methanol. In US-A-4 282 926 the conversion of methanol over a cobalt molybdate catalyst at temperatures of between 200° C and 455° C is described. The conversion of methanol over nickel-containing catalysts, is described in DE-A-23 41 288. In order to reduce carbon deposition, methane recycle and steam addition to the reaction feed are proposed. These measures bring the reaction feed mixture outside the carbon deposition limits which results in a considerable reduction of catalyst deactivation. An obvious disadvantage, however, is a considerable reduction in the overall (thermal) process efficiency.

In addition to the above mentioned aims of the invention, it is an aim of our invention to use the same catalyst for the conversion of methanol to a methane-rich gas.

It has been found that the above mentioned objects of the invention can be accomplished in a process for the production of methane rich gases by the conversion of a mixture comprising hydrocarbons, steam, and optionally methanol or methanol and optionally steam by using nickel catalysts on a thermally stable oxidic carrier, characterized in that the metallic nickel particles are chemically bonded to the thermally stable oxidic carrier.

As thermally stable oxidic carriers there can be used according to the invention the compunds, which according to the state of the art are used as carriers for catalysts in many fields. Such carriers have a large specific surface area. Non-restrictive examples for such carriers are alumina, silica, magnesia, silica-alumina, silica-magnesia, zirconia, silica-zirconia, titania, silica-zirconia-titania, crystalline or amorphous alumino silicate molecular sieves and metal phosphates. According to the invention zirconia and MgO, but especially $Al_2O_3$ and $SiO_2$ are preferred.

The element or elements, the oxides of which are used as carriers according to the invention e.g. aluminium or silicon, are defined hereinafter for the sake of simplicity as carrier base element or elements.

If silica is used as a carrier, commercially available products may be used, such as Aerosil (registered trade mark). Other carriers may also be used in the form of their commercially available products.

According to a preferred embodiment of the invention the chemical bond between the metallic nickel particles and the oxidic carrier is provided by an interfacial layer different from the oxidic carrier, which layer consists of a compound containing oxygen and at least one member selected from the group consisting of nickel and the carrier base element or elements.

Generally the interfacial layer consists of
a) a compound containing nickel ions, or
b) a stable non-stoichiometric oxide of the carrier base element or elements, or
c) a compound containing nickel and the carrier base element or elements.

If the carrier is an oxide of a carrier base element or elements, which form(s) only stoichiometric oxides, the interfacial layer contains nickel ions. If, on the other hand, the carrier is an oxide of a carrier base element or elements, which can form a stable non-stoichiometric oxide, the interfacial layer comprises such non-stoichiometric oxides and may in addition comprise nickel ions.

As carrier base element or elements, which form(s) only stoichiometric oxides, preferably at least one member of the group consisting of Si, Al and Mg is used.

The interfacial layer is very thin and usually has a thickness between 0.2 and 10 nm, preferably between 0.5 and 5 nm. In fact this interfacial layer has a thickness of only a few atoms. It is extremely important that essentially all of the metallic nickel particles of the catalyst are chemically bonded to the carrier as described in the above. The metallic nickel particles of the active catalyst do not form a continuous layer, but are individual particles which are chemically bonded to the carrier.

It has been observed that, if the active catalyst contains appreciable amounts of metallic nickel particles which are not chemically bonded to the carrier, these particles will give rise to the formation of carbon. Therefore, it is preferred that in the catalyst used in the process of the invention per $cm^3$ of the catalyst bed there is not more than 0.05 g, preferably not more than 0.03 g and most preferably not more than 0.01 g metallic nickel, which is not chemically bonded to the carrier, e.g. through the interfacial layer.

Preferably the nickel particles size distribution in the catalyst used in the process of the invention is such that less than 10% by volume of the particles is smaller than 2 nm and less than 10 % by volume of the particles is larger than 30 nm.

Nickel is mostly precipitated on the oxidic carriers

in the form of hydroxide or oxide. The hydroxide or oxide is not, however, catalytically active for the above reactions, so that it has to be reduced to nickel metal. The most obvious way of effecting a chemical bond would be partial reduction of the nickel hydroxide or nickel oxide primarily fixed on the carrier. The nickel particle thus formed is chemically bonded by the remaining nickel oxide, which in turn is bonded by the carrier. There is a problem, however, in that the reduction of a nickel oxide particle, once started, tends to proceed fast until the particle is fully reduced to nickel metal. Part of the nickel ions should be supplied in a form which is reducible more slowly than the pure oxide. To achieve this purpose the nickel ions could be reacted with the carrier into a compound, which is less readily reduced or alternatively a carrier could be used, which can be partially reduced. In the latter case reduction of the carrier material produces metal atoms, often alloyed with nickel, which strongly interact with both the nickel and the carrier.

Reaction of at least part of the nickel ions with the carrier material to form a compound which is less readily reduced than the nickel hydroxide or nickel oxide, can be achieved with $SiO_2$ as a carrier. For instance, a suspension of $SiO_2$ in a solution of nickel salt could be taken, to which an alkaline compound is added in excess over the dissolved temperature if needed, before filtering and drying the loaded carrier, the $SiO_2$ carrier reacts to some extent into nickel hydrosilicate. The portion of the $SiO_2$ reacting into hydrosilicate varies with the reactivity of the $SiO_2$ used and

with the excess of alkaline compound used.

A more complete reaction of $SiO_2$ into hydrosilicate is obtained by coprecipitation of nickel and $SiO_2$, as described by Van Eijck, Van Voorthuijsen and Franzen In Rec. Trav. Chim. 70 (1951) p. 793. According to this method a solution of water glass is thoroughly mixed with a solution of nickel salt, resulting in a more or less complete reaction to form nickel hydrosilicate. Generally the nickel hydrosilicate crystallizes into relatively large, plate-shaped crystallites. Reduction of the coprecipitated material produces nickel particles which adhere very well to the remaining $SiO_2$, which still contains nickel ions. Catalysts prepared according to this method are suitable for the process of the invention.

Referring in more general terms to the methods of preparation of the nickel catalysts useful in the process of the invention it has been found that these nickel catalysts are obtainable by one of the following methods, which are given by way of example only. These methods comprise:

a) mixing an aqueous solution of a nickel salt and an aqueous solution of a salt of the base element and raising the pH-value up to a level where the dissolved nickel and base element or elements ions have been precipitated, aging, if required, the precipitate in the solution, and if required hydrothermally treating the solid, drying, calcinating and reducing; or

b) mixing a solution of a nickel salt and a solution of a salt of the base element or elements, the oxide of

which is the thermally stable oxidic carrier, in an aqueous medium with an aqueous solution of an oxalate or formate, separating, drying, calcinating and reducing the precipitate; or

c) suspending in finely divided form the thermally stable oxidic carrier in a dilute solution of a nickelt salt and precipitating a nickel compound at elevated temperature if required and with vigorous agitation by injection of hydroxyl ions below the level of the vigorously agitated suspension or by forming hydroxile ions by a chemical reaction, which is known per se, of compounds contained also in the solution in an amount of 1 to 10 times the amount stoichiometrically required, followed by separating, drying, calcinating and reducing the loaded carrier; or

d) introducing into the suspension of the thermally stable oxidic carrier a nickel salt solution under the surface of the suspension and keeping the pH-value of the suspension between 4 and 7, separating, drying, calcinating and reducing the loaded carrier.

To obtain a catalyst of sufficient large activity per unit volume a nickel loading of at least 5 % by weight based on the total weight of a catalyst is desirable.

The method referred to under c) hereinabove is known as such from DE-C-17 67 202. In this way highly suitable catalysts can be obtained. It has also been stated in DE-C-17 67 202 that nickel

catalysts thus prepared can be used for methanation of the small quantities of carbon dioxide and carbon monoxide which are present in the gas for the synthesis of ammonia. This relates to the methanation of tiny quantities of carbon monoxide and carbon dioxide (about 0.1 % by vol.) present in a hydrogen/nitrogen mixture.
Since $\alpha$-$Al_2O_3$ carriers form chemical bondings with Ni at the surface of the carrier only at relatively high temperatures, two methods are particularly suitable to obtain chemically bonded Ni on $\alpha$-$Al_2O_3$ as a carrier.

1. Homogeneous precipatation of NiOOH onto alumina by disproportionation of cyanate at a temperature below 60° C, subsequent separation and drying of the precipitate, and calcination at a temperature between 850 and 950° C, and subsequent reduction at about 450° C.

2. Preparation of the precipitate of 1) by homogeneous precipitation using urea, under the condition that the "starting" solution of $Ni^{2+}$, in which $\alpha$-$Al_2O_3$ is dispersed, has a pH below 3. The filtered precipitate is further treated as in 1).

In the process according to the invention there are used in the feed mixture hydrocarbons of 2 to 16 C-atoms per molecule.

The temperature of the feed mixture at the beginning of the catalyst bed is kept, between 300 and 380° C, preferably between 320 and 350° C.

When methanol and optionally steam are fed to the catalyst bed, the steam to methanol ratio is less than 0.8, preferably less than 0.2 mole steam/mole methanol. The entrance temperature to the catalyst bed is kept between 150 and 500° C, preferably between 200 and 250° C.

A hydrogen containing gas can be added to the feed mixture in such an amount that the temperature rise over the catalyst bed is between 150 and 300° C, preferably between 200 and 250° C.

Methanol can be present in the feed mixture in a wide range with referance to the hydrocarbons. In practical procedure the methanol in many times is present in the feed mixture in an amount of more than 0.05 moles, preferably more than 0.2 moles of methanol per mol of hydrocarbons. It is possible, that the feed mixture is essentially free of hydrocarbons and comprises methanol and optionally steam, wherein it is preferred that the steam to methanol ratio is less than 0.8, preferably less than 0.2 moles steam per mol methanol. The ratio of steam to hydrocarbons, preferably, if no methanol is present, is, calculated as moles of steam per carbon atom of the hydrocarbons in the feed mixture below 1.35, preferably below 1.3, and more preferably below 1.2. This ratio should be above 0.8, preferably above 0.9 and more preferably above 1.

Because the nickel particles need to be individually chemically bonded to the support a carrier material has to be used that has a large specific surface area together with a large reactivity. The maximum obtainable loading of chemically bonded nickel depends on the reactivity and the specific surface area.

In particular, it was found that $Ni/SiO_2$ catalysts are highly suitable for the process of the invention.

The presence of an interfacial layer containing nickel ions in the active catalyst can be established in several ways. The oxidation state of nickel in catalysts of this type is normally II. According to one method, first the total nickel content of the catalyst is determined, e.g. by means of atomic absorption spectrometry. Next, the amount of metallic nickel per unit weight of catalyst can be found by measuring the saturation magnetisation of the catalyst sample. The saturation magnetisation found is a direct measure of the amount of metallic nickel present. The difference between the total amount of nickel and the amount of metallic nickel represents the amount of nickel that is present in the interfacial layer in the form of Ni (II) ions.

Alternatively the nickel content that is present as nickel ions in the interfacial layer can be established by measuring the consumption of hydrogen during reduction of the catalyst precursor in a $H_2$ containing atmosphere at temperatures that are also used during the catalyst operation. Subsequently the consumption measured is compared with the volume of hydrogen that would be required for complete reduction of all nickel ions present. From the difference between the two values found the amount of Ni (II) ions can be calculated.

A third way to establish the presence of Ni (II) ions in the active catalyst is to measure the increase in weight of a catalyst sample, that previously has been reduced under reaction conditions, during reoxidation of the sample in a $O_2$ containing atmosphere. When after reduction part of the nickel is present in the interfacial layer in the form of Ni (II) ions the uptake of $O_2$, and hence the increase in weight, will be smaller during reoxidation than when after reduction all the nickel has been reduced. Comparison of the actual increase in weight with the theoretical increase calculated from the total amount of nickel in the sample yields information on the amount of nickel that is present as Ni (II) ions in the interfacial layer in the active catalyst under reaction conditions.

Catalysts used in this invention have preferably nickel ions in the interfacial layer in an amount of at least 5 % preferably at least 10 %, more preferably at least 20 % by weight of the total amount of nickel in the catalyst. The upper limit should be 60 %, preferably 50 % more preferably 40 % by weight.

In the above description two methods have been mentioned to achieve a strong ineraction between the nickel particles and the carrier material. In one of these methods use is made of a carrier, which is to be reduced partially. The compounds or metal atoms produced in reducing the carrier show strong interaction with both the nickel particles and the carrier. An example of such a carrier is $TiO_2$. When heated in a reducing gas $TiO_2$ is reduced to a $TiO_x$-oxide when x has a

value smaller than 2. The oxide reduced at the interface of the nickel particles ensures a strong bond of the nickel particle to the carrier, provided the nickel has been deposited homogeneously over the support and in intimate contact with it. When catalysts are prepared as described in FR-A-2 347 326 by impregnation, drying and subsequent pretreatment the active material is inhomogeneously distributed over the support. Especially at more elevated metal loadings this leads to large clusters of nickel particles only some of which are chemically bonded to the support. The interaction of these "loose" particles with the support remains small even after partial reduction of the carrier. Therefore catalysts prepared according to FR-A-2 347 326 are not suitable for the process according to the invention.

The presence of a partially reduced carrier under conditions of the reaction can be established in the active catalyst in 2 ways. According to one method the metallic nickel content of the catalyst is determined by measuring the saturation magnetisation of a sample of known weight, which previously has been reduced under reaction conditions. Afterwards the sample is reoxidised in an $O_2$ containing atmosphere and simultaneously the increase in weight owing to oxidation of the metallic nickel and, if partially reduced, of the carrier is measured. By comparing the actual increase in weight with the increase that would be expected from oxidation of the metallic nickel only, the extent of reduction of the support can be calculated. In the catalysts used in this invention the increase in weight during oxidation should exceed the increase exclusively caused

by oxidation of the nickel metal by at least 10 %, preferably by at least 20 %.

Alternatively, the extent of reduction of the carrier can be determined by measuring the consumption of $H_2$ during reduction of the completely re-oxidised catalyst in a $H_2$-containing atmosphere at temperatures that are also used in the process of this invention. Subsequently, the consumption measured is compared with the amount of $H_2$ that would be required to yield the amount of nickel which eventually is present as metallic nickel particles in the reduced catalyst. The amount of metallic nickel in the catalyst can be calculated, as indicated above, from a measurement of the saturation magnetisation. From the difference between the actually measured $H_2$-volume and the volume exclusively required for reduction of the nickel in the oxidised form, the extent of reduction of the support can be determined.

In the catalysts used in this invention the $H_2$ consumption during reduction of the catalyst should exceed the consumption exclusively required for reduction of the oxidic nickel by at least 10 %, preferably by at least 20 %.

The nickel particle size distribution can be determined by electron microscope study. Another, faster and more accurate method of determining the particle size distribution is by means of magnetism.

Magnetization is measured as a function of the field strength and the particle size distribution is so computed that the experimental magnetization

curve is reproduced. The computed curve produces an unambiguous particle size distribution, corresponding with the distribution found by means of electron microscope examination.

The process of the invention, largely described in the state of the art, is performed in the following way.
The installation consists of three sections:

1. A first section in which hydrocarbons are treated with hydrogen in a fixed-bed reactor over a cobalt-molybdate catalyst at a temperature of between 300 and 400° C, to convert the organic sulphur compounds present into hydrogen sulphide.

2. A second section in which the hydrogen sulphide formed is absorbed over a zinc oxide bed in a fixed-bed reactor at a temperature between 300 and 350° C. The hydrogen sulphide is converted to steam in this section.

3. A third section, which is the actual low-temperature reforming section, where steam and optionally a hydrogen containing gas are added to the treated hydrocarbon feed and then led to a fixed-bed reactor filled with the catalyst of the invention, after optional additional preheating to a temperature of between 300 and 450° C, to form a methane rich gas which can then be further processed.

Typical product gas compositions have been given in the literature cited before. In compliance with the state of the art, it is also possible

to perform the low temperature reforming step in a split-bed operation as described in DE-A-23 14 804. Likewise, the process of EP-A-0028835 is embodied in our present invention. An additional advantage is that the process of the invention can work with gases with a relatively low $H_2/CO$ ratio in the first stage of the process of EP-A-0028835, as can be concluded from the disclosure of NL-A-82 005 44.

Another option with regard to the above process description is the use of an $Fe_2O_3/SiO_2$ absorption mass instead of the zinc oxide bed. This absorption mass has been described in DE-A-31 31 257 and 32 28 481. It has the great advantage that it can be regenerated, as opposed with ZnO (which must be disposed of after use), while it fulfills the process requirements of having a sulphide "end concentration" in the ppm range, as is required for the downstream reforming step. The latter option would mean a considerably more economic process performance when the sulphur content of the feedstock is relatively high as is the case with certain heavier oil fractions. If, according to the invention, methanol is converted, without the presence of hydrocarbons, the hydrosulphurisation step and the zinc absorbent step can be omitted, since sulphides will mostly not be present in substanial amounts. Meeting the appopriate process requirements is well known in the state of the art so that we need not explain this further.

It is stronghly indicated by our experiments that in the use of nickel catalysts for the conversion of hydrocarbons or methanol as described hereinbefore, deactivation of these catalysts was caused

by the formation of carbon between the nickel-particle and the carrier. This carbon grows into thread-like bodies (so-called whiskers) which push the nickel particles away from the carrier. It turned out that this phenomenon does not take place if the nickel is chemically bonded to the carrier surface.

It was found that only a small amount of non-chemically bonded nickel can be allowed in the catalysts used in this invention. This amount should not exceed 50 mg, more preferably 30 mg and most preferably 10 mg per $cm^3$ reactor volume, as pointed out above already. When the amount of unbonded nickel exceeds such amounts the catalyst bed will gradually be plugged due to the growth of carbon whiskers.

It was established, that very large nickel particles of dimensions of about 100 nm are slowly deactivated under reaction conditions. Nickel particles of such a large size often contain grain boundaries. It is known (Carbon (1972), pages 601 to 611) that at these grain boundaries carbon can be deposited due the which the particles are broken up. The growth of carbon whiskers continues after the nickel particles have been fragmented, which eventually results in reactor plugging. However, nickel

particles of dimensions smaller than 30 nm generally do not contain grain boundaries. Hence, whisker growth induced by grain boundaries will not occur in these particles. Therefore, the catalyst should contain essentially no particles having a particle size over 30 nm.

When carbon is intentionally deposited on a catalyst used according to the present invention by passing pure CO over the catalyst at 275° C, and the carbon is then removed by heating the catalyst in a hydrogen stream at temperatures up to 450° C, it appears that the nickel particle size distribution has not significantly changed. This proves that no carbon has been deposited between the nickel particles and the carrier. In contrast it has been found that in regenerating conventional catalysts, in which carbon has been deposited between the nickel particles and the carrier, far bigger nickel particles are formed than the particles that were present in the original catalyst.

According to the invention, essentially all the metallic nickel particles must be chemically bonded to the carrier particles. It was observed, that when an appreciable amount of the metallic nickel particles is not chemically bonded to the carrier, these "loose" nickel particles will cause whisker carbon formation, leading to catalyst deactivation and reactor plugging.

The relative amount of particles, bonded to the carrier via an interfacial layer can be determined in the following three ways:

0135729

1. A sample of the reduced, active catalyst is investigated in the electron microscope, in order to count the number of metallic nickel particles per gram of catalyst as well as their average size. A similar sample is treated with gaseous pure CO at ambient atmosphere at a temperature between 60 and 100° C for such a time period as is required to remove all metallic nickel from the catalyst - by formation of gaseous Ni $(CO)_4$. Thus only the nickel that is present as ionic nickel in the interfacial layers remains in the catalyst. By electron microscopic investigation the number of interfacial layers - of roughly the same surface size as the original metallic particles - can be counted per gram of catalyst. For a catalyst according to the invention, this number should essentially equal the number, found for the number of metallic nickel particles per gram of catalyst. From the differences between the number of nickel metal particles and the number of nickel interfaces, the amount of "free" or "loose" nickel particles and the weight quantity of "loose" metallic nickel can be calculated.

2. A sample of the reduced, active catalyst is treated with a (gas) mixture of 90 % $N_2$ and 10 % CO at normal pressure at a temperature of 400° C for a time period of between 10 and 60 hours. The sample is then cooled down and passivated. Electron microscopic investigation will then show, depending on whether the catalyst is suitable according to the invention, or not, that carbon whiskers have been formed. These carbon whiskers are only formed at

"loose" metallic nickel particles which are not bonded to the carrier. Thus, the amount of these unbonded particles per gram of catalyst material can be found.

3. For the case that the carrier material consists of an oxide of a base element which is partially reducible, the unbonded nickel particles can be quantitatively determinded by currently available electon microscopic methods.

The use of catalysts, prepared according to DE-C-17 67 202, for the reforming of hydrocarbons with steam, optionnally with the addition of methanol, or for the conversion of methanol directly, optionally with steam addition has never been suggested. It is surprising that, when using these catalysts, catalyst-deactivation and carbon deposition are so much reduced as compared with catalysts according to the state of the art. Generally a marked production of non-reduced nickel atoms is considered to be unfavourable, since nickel is the active component.

It is even more surprising that catalyst deactivation does not occur at steam-to-hydrocarbon ratios which are within the thermodynamic carbon deposition area.

Under conditions that carbon deposition is inevitable, our catalysts exhibit still another attractive property, namely the fact that they can be savely "regenerated" or "re-activated" with oxygen and subsequently with $H_2$.

This is quite remarkable

since reforming catalysts according to the state of the art cannot be regenerated. Obviously this presents considerable technical advantages.

In view of the low tendency of our catalysts for carbon-formation, it proved possible to employ them in low-temperature reforming of hydrocarbons, with steam-to-carbon ratios of less than 0.9, even 0.8, and even less than 0.7 (mole steam/atom carbon of the hydrocarbons). In processes according to the state of the art, the steam-to-carbon ratio (mole/atom) is mostly larger than 1.2, since catalyst deactivation is quite rapid at lower steam to carbon ratios.

The fact that we can use lower steam-to-hydrocarbon ratios allows smaller catalyst volumes and consequently smaller reactors at equal space velocities.

The fact that our catalyst can be used in the direct conversion of methanol without steam and without measurable deactivation over time perios of 500 - 1000 hours, is also quite surprising. This gives a great flexibility as to the feed employable in a process for methane production from liquid feedstocks.

Another interesting phenomenon is the high catalyst activity at already low temperatures. Whereas most commercial catalysts are active for the steam reforming of hydrocarbons at temperatures above 350° C, in most cases even above 400° C, our catalyst has already a considerable activity at a temperature of 300° C. This is shown in the examples.

Still another advantageous property of our new type of catalyst is the fact that the activation procedure, i.e. the reduction of the pre-reduced and subsequently passivated catalyst with hydrogen, is not very critical. It can be performed at relatively high temperature with a gas containing a high hydrogen partial pressure. Obviously this reduces the start-up time of the process which is very favourable. The activation procedure is described in preparation example 2.

It should be noted that in EP-A-0028835 a two stage process for the production of methane has been described, in the first stage of which a mixture containing 25 to 90 % by vol. of hydrogen as well as carbon monoxide and carbon dioxide is passed over a nickel catalyst at a temperature of from 250° C bis 550 ° C to produce a methane rich gas product. For this process the nickel catalyst described in DE-C-17 67 202 may be used, with alumina as the oxidic carrier.

However, this is not an embodiment of the present invention, since by this method no catalyst with chemically bonded nickel will be obtained. This would require a special preparation procedure as mentioned previously.
As is described in the previous state of the art (EP-A-0028835), the addition of hydrogen-rich gas to the reactor feed will reduce catalyst deactivation. Though in our catalyst, deactivation due to carbon deposition is much reduced as compared with conventional catalysts, the presence of hydrogen-rich, or more general,

hydrogen-containing gases may be technologically attractive. An advantageous property of our catalyst still to be mentioned for such case, is the fact that it allows a considerable temperature rise over the catalyst bed of over 200° C, even over 300° C, so that, when the temperature at the entrance of the catalyst bed is e.g. 350° C the reactor exit temperature is allowed to amount to 550° C or even, when the entrance temperature is e.g. 300° C, the exit temperature can amount to 600° C, by virtue of the hydrogen present invoking exothermal hydrogenation reactions.

In view of the above explanation the present invention also covers a process for regenerating a nickel catalyst for use in the production of methane rich gases by the conversion of a mixture comprising hydrocarbons, steam, and optionally methanol or of methanol and optionally steam, characterized in that the nickel catalyst is a catalyst in which the metallic nickel particles are chemically bonded to a thermally stable oxidic carrier and that it is regenerated by the following steps:

a) the catalyst is retained in the reactor in which it has been used and has lost its original activity due to carbon deposition, and the feed gas mixture is shut,
b) the reactor is purged with an inert gas, preferably nitrogen, until essentially no feed gas components or gaseous reaction products remain in the reactor,
c) the carbon deposits are burnt by feeding to the reactor an oxygen containing gas and limiting the maximum temperature in the reactor at 600°C,

d) the reactor is purged with an inert gas, preferably nitrogen, until essentially no oxygen remains in the reactor,

e) the catalyst is reduced with hydrogen at temperatures from 200 to 450°C to its active form.

In step c) the max. temperature is preferably below 550°C or even below 500°C.

The invention also covers a regenerated nickel catalyst obtainable by such process, and it covers the use of such reactivated nickel catalyst for catalysing the conversion of a mixture comprising hydrocarbons, steam, and optionally methanol or of methanol and optionally steam to produce methane rich gases, as described above in detail.

For the regeneration procedure described above the following parameters are preferred:
Purging is preferably done with nitrogen of ambient temperature so that the temperature in the reactor will decrease during the purging step. Subsequently, a nitrogen flow containing a minor amount of about 0.01 to 1, preferably about 0.1% oxygen is fed to the reactor at about ambient temperature leading to (partial) passivation of the catalyst. The oxygen concentration is then gradually raised to a maximum concentration of about 20% (air) in order to avoid any hotspots due to the oxidation reaction. The temperature in the reactor should be kept below 600°C preferably below 500°C at all times. Subsequently, the reactor is again purged with nitrogen in order to remove any oxygen present after which the reactivation procedure which is essentially similar to the original activation procedure of the catalyst using hydrogen containing gas is performed. This is preferably done at a temperature between 200 and 450°C, more preferred between 400 and 450°C.

Preparation example 1.

43.6 g of $Ni(NO_3)_2 \cdot 6H_2O$/ (8.8g metallic nickel) was dissolved in 1 litre of water in which 9.0 g $SiO_2$ ("Aerosil"; trade mark of Degussa), having a surface area of 380 $m^2$/gram had been suspended. The temperature of the vigorously stirred suspension was then raised to 90° C, and the solution was acidified dropwise with nitric acid, until a pH of 2 was attained.

Subsequently 27.0 g of urea was added, which resulted in a gradual rise of the pH, whereby nickel was deposited on the silica in (hydro)oxidic form. The loaded carrier was then separated from the liquid, dried at 120° C, pressed to pellets, and then cut into pieces ranging from 1.5 to 2.5 mm. Next, the catalyst pieces where dehydrated in a nitrogen stream at a temperature up to 450° C and then reduced in a stream of 10 % hydrogen in argon for at least 80 hours. Before the execution of the tests as described in the examples following hereafter, the catalyst was freed from absorbed hydrogen in a nitrogen stream at 450° C for two hours. The nickel particle size distribution of the catalyst thus obtained, is given in table 1.

0135729

Preparation example 2.

This example describes the activation of a so-called passivated catalyst. A catalyst containing a metal as the active component, is in general supplied by the manufacturer in a so-called passivated form. This means, that the catalyst is first completely reduced to form the active metal, and subsequently the catalyst is treated with e.g. diluted air or with $CO_2$, so as to form a very thin oxide layer covering the metal particles, and protecting them against further oxidation. Thus the catalyst can be easily handled and transported, since the reduced catalyst is generally pyrophorous in air, due to the presence of very small metal particles. The latter would be difficult and expensive to handle.

A catalyst sample, prepared according to preparation example 1, incorporating dehydration and reduction, was evacuated for 5 hours at 400° C to achieve complete desorption of the adsorbed hydrogen from the nickel surface. Afterwards, the nickel surface area was measured by hydrogen adsorption at 30° C. At a hydrogen pressure of 40 kPa the uptake of $H_2$ was 25 ml $H_2$ STP/g Ni. Subsequently the catalyst was evacuated at 400° C and passivated at room temperature by passing a flow of 0.5 % $O_2$ at a temperature which gradually rose from 75° C to 300° C in a period of three hours. The catalyst was then kept in the reducing atmosphere at 300° C for another three hours. Then the above mentinned evacuation procedure was again carried out. The subsequent uptake of hydrogen at 30° C and at a hydrogen pressure of 40 kPa was again 25 ml STP/g Ni, indicating that the original nickel surface area was again available after the re-activation procedure of 6 hours.

Example 1:

A cylindrical reactor tube made of stainless steel was filled with a sample of the catalyst prepared and pretreated as described in preparation example 1. The catalyst bed was 1.8 cm in diameter and 17 cm in height. The catalyst sample in the reactor contained 6.3 g of reduced nickel. Before a reaction mixture of 14.3 % $C_4H_{10}$ and 85.7 % $N_2$ entered the reactor, an amount of 6.7 mmol $min^{-1}$ of water was added to this mixture in the preheater kept at 450° C. The temperature at the entrance of the reactor was kept at 400° C. ($H_2O/C=0.8$).

The space velocity in the reactor was 700 $hr^{-1}$ at atmospheric pressure. To establish, whether catalyst plugging (owing to carbon deposition) occurred the pressure drop over the catalyst bed was recorded continuously. The experiment was continued over a period of about 70 hrs. The product gas, the composition of which did not change throughout the experiment, did not contain butane and consisted of 31.9 % $CH_4$, 7.4 % $CO_2$, 0.2 % CO, 2.5 % $H_2$, $H_2O$ (mainly condensed, not determined quantitatively) and $N_2$. During the experiment no increase in pressure drop was observed, indicating the absence of severe filamentous carbon deposition and consequent catalyst plugging.

At the end of the experiment after 70 hrs the temperature at the entrance of the reactor was lowered to 350° C for 4 h. Complete conversion of butane was attained at this temperature. The product gas consisted of 32.4 % $CH_4$, 7.3 % $CO_2$, 0.06 % CO, 1.3 % $H_2$, $H_2O$ (mainly condensed, not determined quantitatively) and $N_2$. After 4 hrs the temperature was further decreased from 350 to 300° C. The conversion of butane

dropped to 6 % only. After 2 h at 300° C the temperature was increased again to 350° C. Whereas butane was completely converted at 350° C before the temperature was lowered to 300° C, afterwards the conversion was limited to 50 % and gradually decreased in 18 h to about 40 %. At a subsequent increase of the temperature to 400° C the conversion rose to 96 %. After another 72 hrs at 400° C the experiment was stopped and the catalyst was subjected to the regeneration procedure described in example 2.

Examle 2:

The catalyst sample, which had been subjected to the reaction sequence described in example 1, was regenerated in the following procedure. The sample was cooled in a $N_2$-stream to 25°C. Next the catalyst was passivated in a mixture of about 0.5 % $O_2$ and 99.5 % $N_2$. Then the catalyst was heated by 50°C $h^{-1}$ from 25 to 450° C in a flow of 20 % $O_2$ and 80 % $N_2$ to burn off the carbon from the nickel surface, which had been deposited owing to the temperature excursion to 300° C, described in the previous example. The temperature was kept constant at 450° C for 5 hrs. Subsequently the sample was cooled down to 100° C in a $N_2$-flow and afterwards heated up again to 450° C in a flow of 10 % $H_2$ and 90 % $N_2$. The heating rate was 50° C $h^{-1}$. After 16 hrs at 450° C in the $H_2/N_2$-mixture the catalyst was freed from adsorbed hydrogen in a flow of nitrogen at 450° C for 2 hrs. Immediately thereafter the experiment described in Example 3 was performed.

Example 3:

The catalyst sample regenerated and re-reduced as described in Example 2 was exposed to a reaction mixture which was obtained as in Example 1 by adding 6.7 mmol $min^{-1}$ of $H_2O$ to a mixture of 14.3 % $C_4H_{10}$ and 85.7 % $N_2$. The temperature at the entrance of the reactor was kept at 350° C. The space velocity in the reactor of a volume of 43.2 $cm^3$ was 700 $h^{-1}$ at atmospheric pressure. The product gas the composition of which did not change throughout the experiment which lasted for 50 hrs, did not contain butane. ($H_2O$/C-ratio was 0.8).

In Example 1 it was seen that prior to regeneration the conversion of butane at 350° C was only 40 %. Hence the absence of butane in the product gas of the regenerated catalyst illustrates the effectiveness of the procedure set out in detail in Example 2. The product gas constisted of 32.4 % $CH_4$, 7.3 % $CO_2$, 0.06 % CO, 1.3 % $H_2$, $H_2O$ (mainly condensed, not determined quantitatively) and $N_2$.

At the end of the successive experiments described in examples 1,2 and 3, with a total duration of about 260 hrs, the pressure drop over the reactor was still negligibly small which indicates that noticeable carbon deposition had not occured.

Example 4:

A 50 wt.%Ni/$SiO_2$-catalyst was prepared according to example 1. The fresh catalyst was dried in air for 24 hrs at 120°C, pressed into tablets (1500 kg $cm^{-2}$, ground and sieved to pellet-size: 0.15 - 0.30 mm. The catalyst was calcined in a 10 % $O_2$/He-stream at 450°C for 16 hrs and reduced in a 10 % $H_2$/Ar-stream for more than 72 hrs at 450°C. The average nickel particle-size of the so prepared catalyst was 6 nm (diameter).

A cylindrical quartz reactor (diameter 1,0 cm) was filled with 2.0 ml of the catalyst. The catalyst bed, containing 0.52g of nickel, exposed some 45 $m^2$ of nickel surface to the gasphase.

A gas mixture containing 7 vol.% of methanol in nitrogen was fed to the reactor at a space-velocity of 1750 $hr^{-1}$. After attainment of the steady-state (typically within 15 min) the product composition was monitored for at least 3 hrs using gaschromatographic analysis. Variations in the steady-state were usually well within 1%. All compounds except water, which was obtained from the mass balance, were measured directly.

As can be seen from Fig. 1 the measured product-compositions are within experimental error (estimated to be about 2 %) equal to the equilibrium-compositions from 367°C onward. The small disrepancy at lower temperatures is due to the incomplete hydrogenation of carbonmonoxide at the mentioned space velocity. Compounds not included in Fig. 1 (e.g. methanol, ethane) were never detected in concentrations above 10 ppm.

In a separate experiment the steady-state composition at 329°C. was established to persist for more than 40 hrs. Moreover, returning to a temperature of 309°C. after about 100 hrs of operation at various temperatures up to 447°C did not change the initial catalyst performance as given by the outlet-composition of Fig. 1.

Example 5

A reactor and catalyst as described in Example 4 were used.

A gasmixture consisting of 5.5 vol. % of methanol ans 10 vol. % of hydrogen, balanced with nitrogen, was fed to the reactor containing 2 ml of the catalyst at a space-velocity of 1750 $hr^{-1}$.

The reactor outlet compositions, compiled in Fig. 2, show that adding hydrogen to the feed also results in equilibrium-compositions for temperatures of 368°C and higher. Small differences with equilibrium-compositions at lower temperatures are again attributed to incomplete hydrogenation of carbonmonoxide at the space-velocity used.

Example 6

A sample of the catalyst prepared and pretreated according to Preparation Example 1 was put into the reactor described in Example 1.

The sample contained 5.6 g of metallic nickel. At a reactor entry temperature of 400°C the catalyst was exposed to a reaction mixture, which was composed of $C_4H_{10}$ (2.1 mmol $min^{-1}$), $H_2O$ (steam) (11.2 mmol $min^{-1}$) and $N_2$ (12.4 mmol $min^{-1}$). The steam to carbon ratio was 1.35. The space velocity applied was about 750 $h^{-1}$ at atmospheric pressure. The absolute pressure in the reactor was 120 kPa. During the experiment the temperature in the catalyst bed was measured at 9 equidistant heights along the axis of the reactor. Moreover, to establish whether reactor plugging due to filamentous carbon deposition occurred, the pressure drop across the catalyst bed was recorded continuously.

It was found that the butane was converted completely. After condensation of most of the water unreacted the product gas was composed of 31.3% $CH_4$, 7.6% $CO_2$, 3.7% $H_2$, 0.07% CO, $H_2O$ (mainly condensed; not determined quantitativel and $N_2$. During the experiment, which lasted for 450 hr, the temperature profile through the catalyst bed did not change markedly. Moreover, at the end of the experiment the pressure drop across the reactor was still negligibly small, which proves the absence of reactor plugging due to carbon deposition on the catalyst. This conclusion was confirmed by examination of the spent catalyst in the electron microscope, which revealed that the catalyst did not contain carbon filaments at all.

Example 7

A sample of the catalyst prepared and pretreated according to Preparation Example 1 was put into the reactor described in Example 1.

The sample contained 5.1 g of metallic nickel. The catalyst was exposed at 400°C to a reaction mixture which was composed of $C_4H_{10}$ (18.6 mmol $min^{-1}$), $H_2O$ (12.2 mmol $min^{-1}$) and $N_2$ (12.4 mmol $min^{-1}$). The space velocity applied was about 1100 $h^{-1}$ at atmospheric pressure. The conversion of butane initially was about 50%, but rapidly decreased to less than 10% after 70 hr. Apparently, the activity of the catalyst could not withstand the extremely small steam-to-carbon ratio of 0.16. After 75 hr the experiment was stopped. During the experiment no increase in pressure drop across the reactor was observed. This is in marked contrast with the performance of other catalysts. It is known from the state of the art, that steam absence, or too low steam concentrations will result in rapid carbon deposition over normal nickel catalysts in a "hydrocarbon atmosphere" leading to reactor plugging within less than a few hours. Examination of the spent catalyst in the electron microscope revealed that only very few carbon filaments of diameters of about 10 nm had been formed. As will be shown in Example 8 catalysts according to the invention thus deactivated can regain their original activity by a treatment in air at elevated temperatures. Processes of this type are always designed for such operating conditions that an uninterupted production can be guaranteed for several thousand hours. However, there may arise emergency situations due to sudden blockage of steam supply for which emergency procedures are always taken into the design. In these procedures the performance of the catalyst is of big importance. If the catalyst is very vulnerable to the possibility of carbon

deposition then much damage will be done in an emergency case. Therefore, emergency procedures designed for such cases will be very strict. On the other hand, if the catalyst is less vulnerable for such emergeny cases because carbon deposition will not occur very rapidly as we have shown now for our catalyst, then the emergency procedures may be less strict with obviously economic advantages.

Example 8

A sample of the catalyst prepared and pretreated according to Preparation Example 1 was put into the reactor described in Example 1.

The sample contained 5.0 g of metallic nickel. At a reactor entry temperature of 400$^oC$ the catalyst was exposed to a reaction mixture, which was composed of $C_4H_{10}$ (4.1 mmol $min^{-1}$), $H_2$ (1.3 mmol $min^{-1}$), $H_2O$ (13.9 mmol $min^{-1}$) and $N_2$ (6.1 mmol $min^{-1}$). The steam to carbon ratio was 0.85. The space velocity applied was about 730 $h^{-1}$ at atmospheric pressure. During the experiment the temperature in the catalyst bed was measured at 9 equidistant heights along the axis of the reactor. In addition to establish whether reactor plugging owing the filamentous carbon deposition occurred, the pressure drop across the catalyst bed was recorded continuously.

It was found that initially the butane was converted completely. After condensation of most of the water unreacted the product gas was composed of 59.4% $CH_4$, 13.6% $CO_2$, 6.6% $H_2$, 0.07% CO, $H_2O$ (mainly condensed; not determined quantitatively) and $N_2$. From the temperature profile measured in the catalyst bed it was obvious that the reaction zone gradually passed through the catalyst bed.

Apparently, the activity of the catalyst steadily decreased. Owing to the deactivation butane was present in the product gas in tiny amounts after about 200 h. After 550 h the conversion of butane decreased to only 42%. As the pressure drop across the catalyst bed did not increase at all, it was concluded that extensive filamentous carbon deposition did not occur. Hence, the gradual decrease of the catalytic activity was attributed to the formation of non-filamentous carbonaceous deposits poisoning the nickel surface.

It was tried to reactivate the partially deactivated catalyst by a treatment in air at elevated temperatures, followed by reactivation in a reduction step. To this end the reaction sequence described in detail in Example 2 - which procedure comprises passivation, oxidation and re-reduction - was carrier out in situ. Afterwards the reaction mixture was passed once more through the catalyst bed under identical conditions as described above. It was established that butane was again converted completely. After another 200 hr the conversion of butane was still virtually complete. Then the catalyst was deactivated owing to maloperation causing a high partial pressure of butane in the catalyst bed for several hours. The deactivated catalyst was reactivated for the second time in exactly the same way as before. Subsequently the reaction mixture was again passed through the catalyst bed. For about 200 h after the restart of the experiment the temperature profile in the catalyst bed hardly changed. Afterwards a gradual deactivation of the catalyst was apparent from a continuous shift of the temperature profile through the catalyst bed. At about 400 h after the (second) restart the experiment was stopped. At that time the conversion of butane was still above 99.9%. The pressure drop across the catalyst bed was still as small as at the very beginning of the experiment indicating the absence of extensive filamentous carbon deposition.

Table 1

| Fraction, % by vol.: | Diameter, nm |
|---|---|
| 0 | < 2.5 |
| 3.40 | 2.69 |
| 5.30 | 2.90 |
| 2.89 | 3.13 |
| 7.17 | 3.37 |
| 10.67 | 3.63 |
| 6.44 | 3.91 |
| 9.12 | 4.22 |
| 3.20 | 4.54 |
| 1.60 | 4.90 |
| 7.33 | 5.27 |
| 4.28 | 5.68 |
| 8.43 | 6.12 |
| 5.68 | 6.60 |
| 7.15 | 7.11 |
| 5.10 | 7.66 |
| 3.61 | 8.26 |
| 3.13 | 8.90 |
| 0.00 | 9.58 |
| 0.00 | 10.3 |
| 0.00 | 11.1 |
| 4.22 | 12.0 |
| 1.28 | 12.9 |
| 0 | > 13 |

2. Juli 1984

Claims:

1. Process for the production of methane rich gases by the conversion of a mixture comprising hydrocarbons, steam, and optionally methanol or methanol and optionally steam by using nickel catalysts on a thermally stable oxidic carrier, characterized in that the metallic nickel particles are chemically bonded to the thermally stable oxidic carrier.

2. A process as claimed in claim 1, characterized in that the carrier is an oxide of at least one member of the group consisting of Si, Al and Mg.

3. A process as claimed in claim 1, characterized in that the carrier is $SiO_2$.

4. A process as claimed in claim 1, characterized in that the carrier is $Al_2O_3$ or MgO.

5. A process according to any of claims 1 to 4, characterized in that methanol is present in the feed mixture in an amount of more than 0.05 moles, preferably more than 0.2 moles of methanol per mol of hydrocarbons.

6. A process according to any of claims 1 to 4, characterized in that the feed mixture is essentially free of hydrocarbons and comprises methanol and optionally steam, wherein the steam to methanol ratio is less than 0.8, preferably less than 0.2 mole steam per mole methanol.

7. A process according to any of claims 1 to 6, characterized in that the ratio of steam to hydrocarbons, calculated as moles of steam per moles carbon atom of the hydrocarbons in the feed mixture is below 1.35, preferably below 1.3, more preferably below 1.2.

8. A process according to any of claims 1 to 6, characterized in that the ratio of steam to hydrocarbons, calculated as moles of steam per moles carbon atom of the hydrocarbons in the feed mixture is above 0.8, preferably above 0.9, more preferably above 1.

9. A process according to any of claims 1 to 8, characterized in that the nickel catalyst is obtainably by

a) mixing an aqueous solution of a nickel salt and an aqueous solution of a salt of the base element and raising the pH-value up to a level where the dissolved nickel and base element or elements ions have been precipitated, aging, if required, the precipitate in the solution, and if required hydrothermally treating the solid, drying, calcinating and reducing; or

b) mixing a solution of a nickel salt and a solution of a salt of the base element or elements, the oxide of which is the thermally stable oxidic carrier, in an aqueous medium with an aqueous solution of an oxalate or formate, separating, drying, calcinating and reducing the precipitate; or

c) suspending in finely divided form the thermally stable oxidic carrier in a dilute solution of a nickel salt and precipitating a nickel compound at elevated temperature if required and with vigorous agitation by injection of hydroxyl ions below the level of the vigorously agitated suspension or by forming hydroxile ions by a chemical reaction, which is known per se, of compounds contained also in the solution in an amount of 1 to 10 times the amount stoechiometrically required, followed by separating, drying, calcinating and reducing the loaded carrier; or

d) introducing into the suspension of the thermally stable oxidic carrier a nickel salt solution under the surface of the suspension and keeping the pH-value of the suspension between 4 and 7, separating, drying, calcinating and reducing the loaded carrier.

10. A process for regenerating a nickel catalyst for use in the production of methane rich gases by the conversion of a mixture comprising hydrocarbons, steam, and optionally methanol or of methanol and optionally steam, characterized in that the nickel catalyst is a catalyst in which the metallic nickel particles are chemically bonded to a thermally stable oxidic carrier and that it is regenerated by the following steps:

a) the catalyst is retained in the reactor in which it has been used and has lost its original activity due to carbon deposition, and the feed gas mixture is shut,

b) the reactor is purged with an inert gas, preferably nitrogen, until essentially no feed gas components or gaseous reaction products remain in the reactor,

c) the carbon deposits are burnt by feeding to the reactor an oxygen containing gas and limiting the maximum temperature in the reactor at 600°C,

d) the reactor is purged with an inert gas, preferably nitrogen, until essentially no oxygen remains in the reactor,

e) the catalyst is reduced with hydrogen at temperatures from 200 to 450°C to its active form.

11. A process according to claim 10, characterized in that the carrier is an oxide of at least one member of the group consisting of Si, Al and Mg.

12. A process according to claim 10, characterized in that the carrier is $SiO_2$.

13. A process according to claim 10, characterized in that the carrier is $Al_2O_3$ or MgO.

14. A process according to any of claims 10 to 13, characterized in that the nickel catalyst is obtainable by

a) mixing an aqueous solution of a nickel salt and an aqueous solution of a salt of the base element and raising the pH-value up to a level where the dissolved nickel and base element or elements ions have been precipitated, aging, if required, the precipitate in the solution, and if required hydrothermally treating the solid, drying, calcinating and reducing, or

b) mixing a solution of a nickel salt and a solution of a salt of the base element or elements, the oxide of which is the thermally stable oxidic carrier, in an aqueous medium with an aqueous solution of an oxalate or formate, separating, drying, calcinating and reducing the precipitate; or

c) suspending in finely divided form the thermally stable oxidic carrier in a dilute solution of a nickel salt and precipitating a nickel compound at elevated temperature if required and with vigorous agitation by injection of hydroxyl ions below the level of the vigorously agitated suspension or by forming hydroxile ions by a chemical reaction, which is known per se, of compounds contained also in the solution in an amount of 1 to 10 times the amount stoechiometrically required, followed by separating, drying, calcinating and reducing the loaded carrier; or

d) introducing into the suspension of the thermally stable oxidic carrier a nickel salt solution under the surface of the suspension and keeping the pH-value of the suspension between 4 and 7, separating, drying, calcinating and reducing the loaded carrier.

15. A regenerated nickel catalyst useful for catalysing the conversion of a mixture comprising hydrocarbons, steam, and optionally methanol or of methanol and optionally steam to produce methane rich gases, characterized by the fact, that the nickel catalyst is a catalyst in which the metallic nickel particles are chemically bonded to a thermally stable oxidic carrier and which, after it lost its original activity in the said conversion, is obtainable in a reactivated form by the following steps:

a) the catalyst is retained in the reactor in which it has been used and has lost its original activity due to carbon deposition, and the feed gas mixture is shut,

b) the reactor is purged with an inert gas, preferably nitrogen, until essentially no feed gas components or gaseous reaction products remain in the reactor,

c) the carbon deposits are burnt by feeding to the reactor an oxygen containing gas and limiting the maximum temperature in the reactor at 600°C,

d) the reactor is purged with an inert gas, preferably nitrogen, until essentially no oxygen remains in the reactor,

e) the catalyst is reduced with hydrogen at temperatures from 200 to 450°C to its active form.

16. Use of a reactivated nickel catalyst for catalysing the conversion of a mixture comprising hydrocarbons, steam, and optionally methanol or of methanol and optionally steam to produce methane rich gases, which catalyst is a catalyst in which the metallic nickel particles are chemically bonded to a thermally stable oxidic carrier and which, after it lost its original activity in the said conversion, has been reactivated, such reactivated form being obtainable by the following steps:

a) the catalyst is retained in the reactor in which it has been used and has lost its original activity due to carbon deposition, and the feed gas mixture is shut,

b) the reactor is purged with an inert gas, preferably nitrogen, until essentially no feed gas components or gaseous reaction products remain in the reactor,

c) the carbon deposits are burnt by feeding to the reactor an oxygen containing gas and limiting the maximum temperature in the reactor at 600°C,

d) the reactor is purged with an inert gas, preferably nitrogen, until essentially no oxygen remains in the reactor,

e) the catalyst is reduced with hydrogen at temperatures from 200 to 450°C to its active form.

0135729



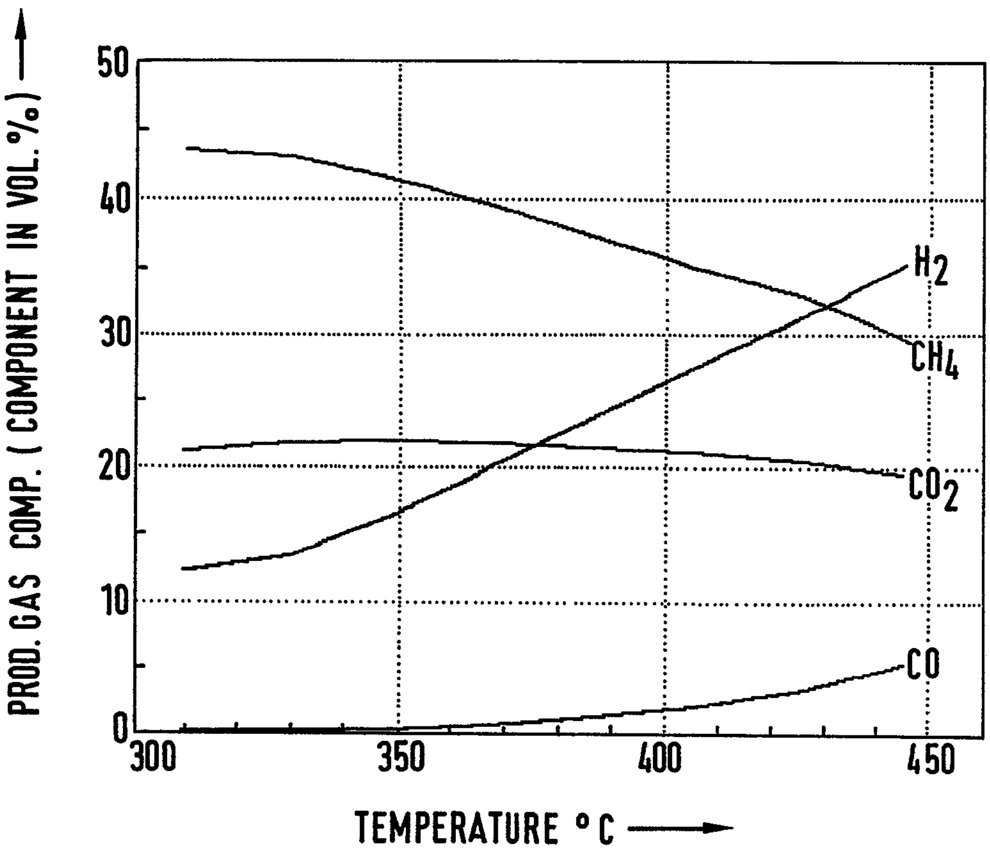


FIG.1

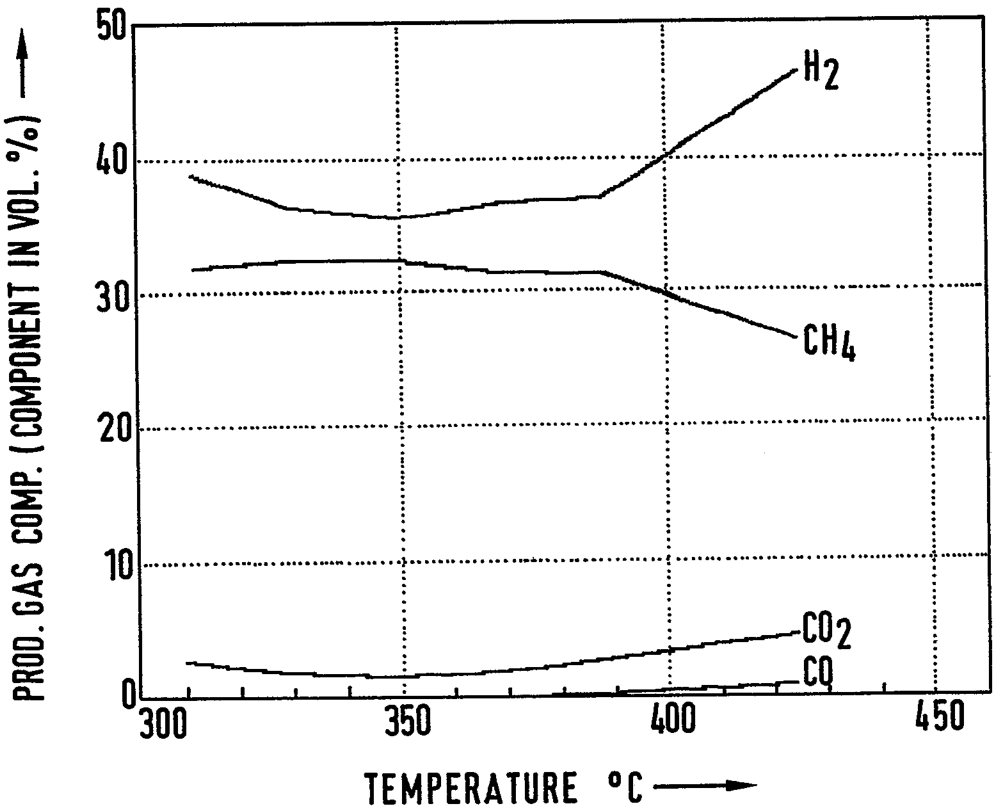
50
40
30
20
10
0
300
350
400
450
PROD. GAS COMP. (COMPONENT IN VOL. %)
TEMPERATURE °C
H2
CH4
CO2
CO

FIG.2

European Patent Office

# EUROPEAN SEARCH REPORT

0135729

Application number

EP 84 10 9159

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | EP-A-0 028 835 (VEG-GASINSTITUUT) * examples; claims; page 6, paragraphs 3-4 * | 1,2,4 | C 07 C 9/04<br>C 07 C 4/06<br>C 07 C 1/20<br>B 01 J 23/74<br>B 01 J 23/94 |
| P,X | EP-A-0 086 538 (VEG-GASINSTITUUT) * claims * & NL - A - 8 200 544 (Cat. D) | 9,14 | |
| D,X | DE-A-1 767 202 (STAMICARBON) * claims * | 9-14 | |
| X | EP-A-0 003 737 (METALLGESELLSCHAFT) | 1 | |

TECHNICAL FIELDS SEARCHED (Int. Cl.4)

C 07 C 9/00
C 07 C 4/00
C 07 C 1/00

The present search report has been drawn up for all claims

| Place of search | Date of completion of the search | Examiner |
|---|---|---|
| THE HAGUE | 26-10-1984 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document